# EUROPEAN PATENT APPLICATION

(11) **EP 4 449 904 A1**
(43) Date of publication of application: **23.10.2024**
(21) Application number: 22914966.1
(22) Date of filing: 28.12.2022
(51) Int. Cl.: A24F 40/10

(54) **AEROSOL SUPPLY SYSTEM AND ATOMIZER THEREOF**

(30) Priority: 30.12.2021 CN 202111654469
(71) Applicant: Shenzhen First Union Technology Co., Ltd., Shenzhen, Guangdong 518000 (CN)
(72) Inventor: HU, Ruilong, Shenzhen, Guangdong 518000 (CN); GUO, Zhiwei, Shenzhen, Guangdong 518000 (CN); XU, Zhongli, Shenzhen, Guangdong 518000 (CN); LI, Yonghai, Shenzhen, Guangdong 518000 (CN)
(74) Representative: Ran, Handong
(86) International application number: PCT/CN2022/142986
(87) International publication number: WO 2023/125715

(57) **Abstract**

An aerosol supply system (100) and an atomizer (100A) used therein, which relate to the technical field of electronic atomization. The aerosol supply system (100) is configured as atomizing a liquid substrate to generate an aerosol, and comprises a liquid storage cavity (10A), an electromagnetic induction coil (60), an induction heating body (20), and a capillary element (30); the liquid storage cavity (10A) is used for storing the liquid substrate; the electromagnetic induction coil (60) is used for generating a variable magnetic field; the induction heating body (20) is used for induction heating in the variable magnetic field; the capillary element (30) is arranged as in contact with the induction heating body (20) and is used for receiving and storing the liquid substrate from the liquid storage cavity (10A) and conveying the liquid substrate to the vicinity of the induction heating body (20); and the capillary element (30) is positioned between the electromagnetic induction coil (60) and the induction heating body (20). By the described means, the aerosol supply system (100) does not need to be provided with an electrical conduction structure such as an electrode lead for establishing an electrically conductive connection to a heating body, which allows quick and easy assembly of the atomizer (100A) comprising the induction heating body (20) and the capillary element (30) in the aerosol supply system (100).

## Description

### CROSS-REFERENCE TO RELATED APPLICATIONS

This application claims priority to Chinese Patent Application No. 202111654469.3, filed with the China National Intellectual Property Administration on December 30, 2021 and entitled "AEROSOL SUPPLY SYSTEM AND ATOMIZER THEREOF", which is incorporated herein by reference in its entirety.

### TECHNICAL FIELD

This application relates to the field of electronic atomization technologies, and in particular, to an aerosol supply system and an atomizer of the aerosol supply system.

### BACKGROUND

An aerosol supply system is an electronic product that heats and atomizes a liquid substrate such as e-liquid or liquid medicine into an aerosol for inhalation.

The aerosol supply system may include an atomizer and a power supply assembly, where the power supply assembly is configured to supply power to the atomizer; and the atomizer may include an atomization core assembly and an atomization chamber, where the atomization core assembly is configured to generate heat when powered on to atomize the liquid substrate, and the atomization chamber is for supplying a liquid substrate to be heated and atomized to the atomization core assembly.

The aerosol supply system usually uses a porous ceramic body as a capillary liquid guiding element for absorbing the liquid substrate, and uses a heating element arranged on an atomization surface of the porous ceramic body to heat at least a part of the liquid substrate in the porous ceramic body to generate an aerosol.

Currently, in an e-liquid atomization technology, ceramic is mostly used to generate heat. In some solutions, a ceramic heating surface is provided upward. In other words, the heating surface is toward a mouthpiece of an atomization device. This needs an electrode pin to be bent at an angle, so that the electrode pin is connected to an electrode of a heating element. However, it is difficult to control the bending angle and elastic force of the electrode pin, making electrode assembly inconvenient, and it is also difficult to control elastic force of the electrode when the electrode is in contact with a ceramic surface. In addition, a ceramic core needs to be sealed with silica gel, so that the ceramic core is sealed and assembled in an atomization seat. However, this makes assembly inconvenient, and a sealing effect is not good, which may result in liquid leakage. In addition, costs of using the ceramic core to generate heat are high. Further, inlet air needs to bypass two side walls of a fixed seat of the ceramic core, making an airflow stroke long.

### SUMMARY

This application aims to provide an aerosol supply system and an atomizer thereof, to resolve a technical problem that an existing aerosol supply system and an atomizer thereof are not easy to assemble.

The following technical solution is used to resolve the technical problem in this application: An aerosol supply system is provided, configured to atomize a liquid substrate to generate an aerosol, where the aerosol supply system includes: a liquid storage cavity, an electromagnetic induction coil, an induction heating body, and a capillary element. The liquid storage cavity is for storing the liquid substrate. The electromagnetic induction coil is configured to generate a variable magnetic field. The induction heating body is configured to induce heating in the variable magnetic field. The capillary element is arranged in contact with the induction heating body, and configured to receive and store the liquid substrate from the liquid storage cavity and convey the liquid substrate to vicinity of the induction heating body. The capillary element is positioned between the electromagnetic induction coil and the induction heating body.

In a preferred embodiment, the induction heating body is a flat extended heating sheet with holes; and/or the electromagnetic induction coil is a coil extending in a plane.

In a preferred embodiment, the aerosol supply system includes an airflow channel, and a part of the airflow channel guides airflow to bypass or penetrate the capillary element and reach an atomization space above the induction heating body.

In a preferred embodiment, the capillary element is positioned further away from the liquid storage cavity than the induction heating body.

In a preferred embodiment, the induction heating body is stacked on an upper surface of the capillary element, and is pressed and fixed on the capillary element through a support.

In a preferred embodiment, the aerosol supply system includes a housing assembly, the housing assembly defines the liquid storage cavity and includes a mouthpiece portion, a bottom end portion, and a smoke exhaust pipe, the bottom end portion is arranged opposite to the mouthpiece portion, and the smoke exhaust pipe extends from the mouthpiece portion toward the bottom end portion in the housing assembly; and the capillary element is located in the housing assembly and arranged on the bottom end portion, and the induction heating body is positioned closer to the smoke exhaust pipe than the capillary element.

In a preferred embodiment, the aerosol supply system further includes a support located in the housing assembly, the support defines a first channel in communication with the liquid storage cavity to guide the liquid substrate to the capillary element and a second channel in communication with the smoke exhaust pipe, and at least a part of the second channel is used as an atomization space above the induction heating body.

In a preferred embodiment, the support includes an elastic pressing member and a supporting component configured to support the elastic pressing member; and the elastic pressing member is configured to press and fix the induction heating body on a surface of the capillary element.

In a preferred embodiment, an air inlet hole is provided on the bottom end portion of the housing assembly, and the air inlet hole extends in a longitudinal direction, so that a port of the air inlet hole is higher than an upper surface of the capillary element; and a notch is provided on the support, so that airflow output through the air inlet hole reaches the atomization space above the induction heating body via the notch.

In a preferred embodiment, the bottom end portion of the housing assembly includes an air inlet post extending through the capillary element, and the interior of the air inlet post is hollow, and a tail end of the air inlet post is higher than an upper surface of the capillary element.

In a preferred embodiment, the induction heating body is pressed and fixed on the capillary element through a support; the housing assembly includes an upper housing and a base, the base includes the bottom end portion, the support is mounted on the base and is used as a second sealing member, and at least a part of the second sealing member is constructed to provide sealing between the base and the upper housing; and the second sealing member and the base enclose a third channel, the third channel is in communication with the liquid storage cavity to guide the liquid substrate to the capillary element, the second sealing member further includes a second channel in communication with the smoke exhaust pipe, and at least a part of the second channel is constructed as an atomization space above the induction heating body.

In a preferred embodiment, the aerosol supply system includes a power supply assembly, the power supply assembly includes a battery housing, a battery, and the electromagnetic induction coil, the battery housing accommodates the battery and the electromagnetic induction coil, and the electromagnetic induction coil is electrically connected to the battery.

In a preferred embodiment, the electromagnetic induction coil and the induction heating body are arranged in parallel.

The following technical solution is used to resolve the technical problem in this application: An atomizer of an aerosol supply system is provided, where the atomizer includes: a housing assembly, defining a liquid storage cavity for storing a liquid substrate, and including a mouthpiece portion, a bottom end portion, and a smoke exhaust pipe, where the bottom end portion is arranged opposite to the mouthpiece portion, and the smoke exhaust pipe extends from the mouthpiece portion toward the bottom end portion in the housing assembly; an induction heating body, configured to be capable of inducing heating in a varying magnetic field, to heat a part of the liquid substrate to generate an aerosol; and a capillary element, arranged in contact with the induction heating body, and configured to receive and store the liquid substrate from the liquid storage cavity and convey the liquid substrate to vicinity of the induction heating body, where the induction heating body is positioned closer to the smoke exhaust pipe than the capillary element.

In a preferred embodiment, the capillary element is located between the induction heating body and the bottom end portion of the housing assembly.

In a preferred embodiment, the induction heating body is stacked on an upper surface of the capillary element, and is pressed and fixed on the capillary element through a support.

In a preferred embodiment, the capillary element is arranged in a flat plate shape, and a thickness of the capillary element ranges from 1 mm to 1.5 mm.

In a preferred embodiment, an air inlet channel is provided on the housing assembly, and the air inlet channel bypasses or penetrates the capillary element, to guide airflow to vicinity of the induction heating body.

Beneficial effects of this application are as follows: In the aerosol supply system and the atomizer in embodiments, an electromagnetic induction coil is used to generate a variable magnetic field to perform induction heating on an induction heating body, so that a conductive structure, for example, an electrode pin, does not need to be arranged for conductive connection to the heating body. This makes it easy and convenient to assemble an atomizer including the induction heating body and a capillary element in the aerosol supply system, and avoids difficulty in bending and assembling an electrode elastic sheet. In addition, because the induction heating body does not need to be connected to a wire, the induction heating body can be arranged further away from the electromagnetic induction coil than the capillary element. In this way, it is convenient to orient a heating surface of the capillary element toward or close to a smoke exhaust pipe of the aerosol supply system, so that an aerosol generated through atomization does not need to bypass the capillary element, but directly enters the smoke exhaust pipe, and is inhaled by a user.

### BRIEF DESCRIPTION OF THE DRAWINGS

One or more embodiments are exemplarily described with reference to corresponding figures in the accompanying drawings, and the exemplary descriptions do not constitute a limitation on the embodiments. Elements in the accompanying drawings that have same reference numerals are represented as similar elements, and unless otherwise particularly stated, the figures in the accompanying drawings are not drawn to scale.
FIG. 1 is a schematic three-dimensional assembly view of an aerosol supply system according to an embodiment of this application;
FIG. 2 is a schematic sectional view of the aerosol supply system shown in FIG. 1;
FIG. 3 is a schematic enlarged view of Part I in FIG. 2;
FIG. 4 is another schematic sectional view of the aerosol supply system shown in FIG. 1;
FIG. 5 is a schematic three-dimensional exploded view of the aerosol supply system shown in FIG. 1;
FIG. 6 is a schematic three-dimensional view of an induction heating body of the aerosol supply system shown in FIG. 5;
FIG. 7 is a schematic three-dimensional view of an electromagnetic induction coil of the aerosol supply system shown in FIG. 5;
FIG. 8 is a schematic three-dimensional view an elastic pressing member of the aerosol supply system shown in FIG. 5;
FIG. 9 is another schematic three-dimensional view of the elastic pressing member shown in FIG. 8;
FIG. 10 is a schematic three-dimensional view of a capillary element of the aerosol supply system shown in FIG. 5;
FIG. 11 is a schematic three-dimensional view of a supporting component of the aerosol supply system shown in FIG. 5;
FIG. 12 is another schematic three-dimensional view of a supporting component shown in FIG. 9;
FIG. 13 is a schematic sectional view of a first sealing member of the aerosol supply system shown in FIG. 5;
FIG. 14 is another schematic three-dimensional view of the first sealing member shown in FIG. 13;
FIG. 15 is a schematic three-dimensional view of a base of the aerosol supply system shown in FIG. 5;
FIG. 16 is a schematic three-dimensional view of an upper housing of the aerosol supply system shown in FIG. 5;
FIG. 17 is a schematic three-dimensional view of a bottom cover of the aerosol supply system shown in FIG. 5;
FIG. 18 is a schematic three-dimensional view of an insulation member of the aerosol supply system shown in FIG. 5;
FIG. 19 is a schematic three-dimensional view of a coil support of the aerosol supply system shown in FIG. 5;
FIG. 20 is a schematic sectional view of an aerosol supply system according to another embodiment of this application;
FIG. 21 is a schematic enlarged view of Part II in FIG. 20;
FIG. 22 is a schematic three-dimensional view of a support of the aerosol supply system shown in FIG. 20;
FIG. 23 is another schematic three-dimensional view of the support shown in FIG. 22;
FIG. 24 is a schematic three-dimensional view of a base of the aerosol supply system shown in FIG. 20;
FIG. 25 is another schematic three-dimensional view of a base shown in FIG. 22; and
FIG. 26 is a schematic three-dimensional view of an electromagnetic induction coil of the aerosol supply system shown in FIG. 20.

### DETAILED DESCRIPTION

For ease of understanding of this application, this application is described below in more detail with reference to the accompanying drawings and specific embodiments. It should be noted that, when an element is expressed as "being fixed to" another element, the element may be directly on the another element, or one or more intermediate elements may exist between the element and the another element. When an element is expressed as "being connected to" another element, the element may be directly connected to the another element, or one or more intermediate elements may exist between the element and the another element. Terms "vertical", "horizontal", "left", "right", "inner", "outside", and similar expressions used in this specification are merely used for an illustrative purpose.

Unless otherwise defined, meanings of all technical and scientific terms used in this specification are the same as that usually understood by a person skilled in the technical field to which this application belongs. Terms used in the specification of this application are merely intended to describe objectives of the specific embodiments, but are not intended to limit this application. A term "and/or" used in this specification includes any or all combinations of one or more related listed items.

In addition, technical features involved in different embodiments of this application described below may be combined together if there is no conflict.

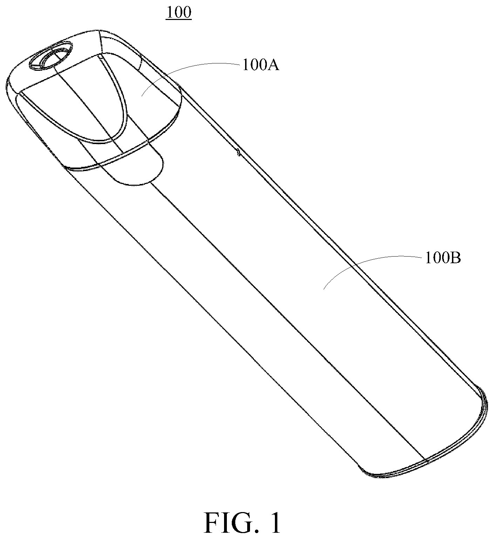

FIG. 1 is a schematic three-dimensional assembly view of an aerosol supply system 100 according to an embodiment of this application. The aerosol supply system 100 is configured to atomize a liquid substrate to generate an aerosol, where the aerosol supply system 100 may include: an atomizer 100A storing and atomizing the liquid substrate to generate the aerosol; and a power supply assembly 100B supplying heating energy to the atomizer 100A. The liquid substrate may be liquid such as e-liquid or liquid medicine. The liquid substrate may also be referred to as liquid, atomization may also be referred to as vaporization, and the aerosol may also be referred to as flue gas, aerial fog, or atomized gas.

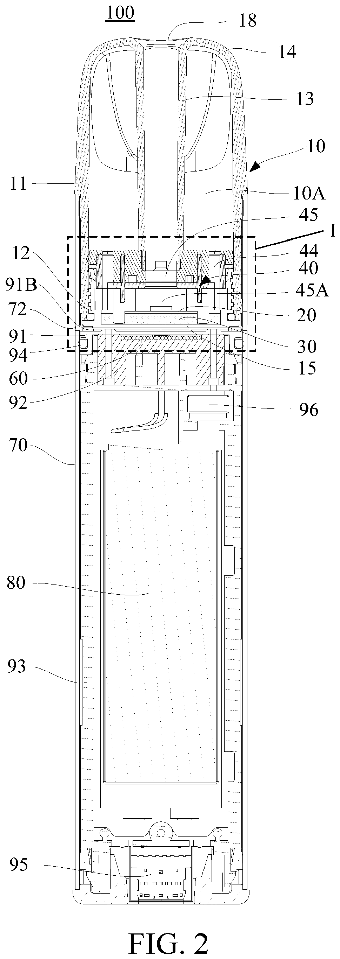

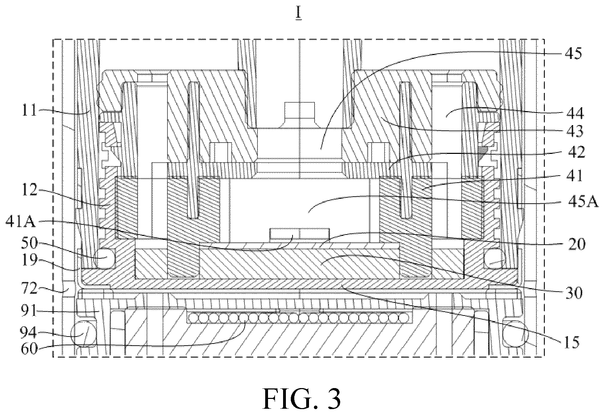

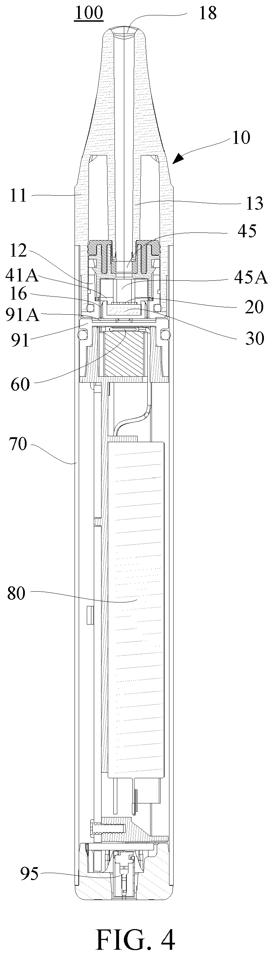

FIG. 2 and FIG. 4 are two schematic sectional views of the aerosol supply system 100 shown in FIG. 1, and FIG. 3 is a schematic enlarged view of Part I in FIG. 2. As shown in the figures, in an embodiment, the aerosol supply system 100 may include a liquid storage cavity 10A, an induction heating body 20, a capillary element 30, and an electromagnetic induction coil 60. The liquid storage cavity 10A is an accommodation space defined in the aerosol supply system 100, for storing the liquid substrate and supplying the liquid substrate to the capillary element 30. The electromagnetic induction coil 60 may be arranged by using a wire, and is configured to generate a variable magnetic field when the electromagnetic induction coil 60 is powered on. The induction heating body 20 may be arranged in the variable magnetic field generated by the electromagnetic induction coil 60, so that the induction heating body 20 can be penetrated by the varying magnetic field to generate heat, to heat the liquid substrate. The capillary element 30 is arranged in contact with the induction heating body 20, and is configured to receive and store the liquid substrate from the liquid storage cavity 10A via a capillary space of the capillary element 30, and convey the liquid substrate to vicinity of the induction heating body 20 through capillary action, so that the capillary element 30 can continuously supply the liquid substrate toward the induction heating body 20 as the induction heating body 20 heats up the liquid substrate close to the induction heating body 20 in the capillary element 30. The capillary element 30 is positioned between the electromagnetic induction coil 60 and the induction heating body 20. For example, the capillary element 30 may be prepared by a material having a capillary channel or pores, for example, a hard or rigid capillary structure such as fibrous cotton, a porous ceramic body, a glass fiber rope, porous glass ceramic, or porous glass. The capillary element 30 is in fluid communication with the liquid storage cavity 10A, to absorb the liquid substrate conveyed from the liquid storage cavity 10A. For example, oil guiding cotton may be used as the capillary element 30 for oil guiding. Because the oil guiding cotton may have a sealing and oil locking capability, the induction heating body 20 may be placed on the capillary element 30, so that an atomization area is increased, and an atomization effect is better.

In the aerosol supply system 100 in this embodiment, an electromagnetic induction coil 60 is used to generate a variable magnetic field to perform induction heating on an induction heating body 20, so that a conductive structure, for example, an electrode pin, does not need to be arranged for conductive connection to the heating body. This makes it easy and convenient to assemble an atomizer including the induction heating body 20 and a capillary element 30 in the aerosol supply system, and avoids difficulty in bending and assembling an electrode elastic sheet. In addition, because the induction heating body 20 does not need to be connected to a wire, the induction heating body can be arranged further away from the electromagnetic induction coil 60 than the capillary element 30. In this way, it is convenient to orient a heating surface of the capillary element 30 toward or close to a smoke exhaust pipe 13 of the aerosol supply system 100, so that an aerosol generated through atomization does not need to bypass the capillary element 30, but directly enters the smoke exhaust pipe 13, and is inhaled by a user.

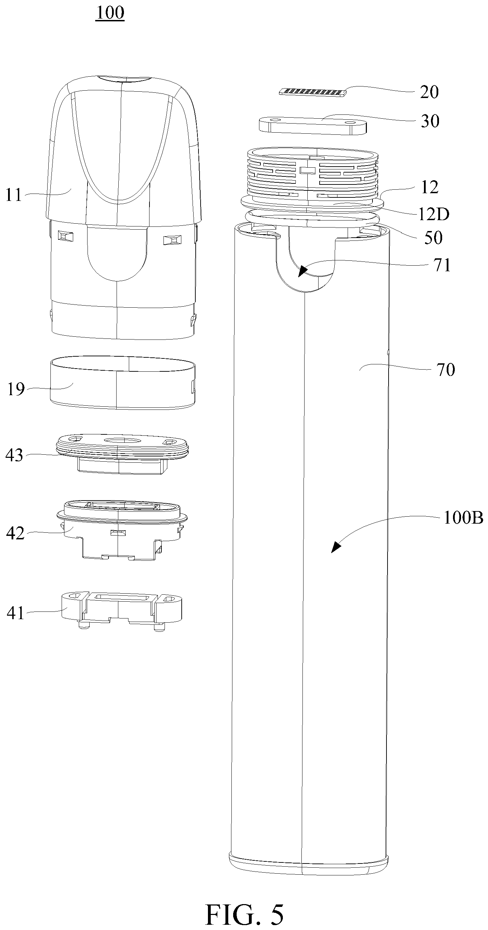

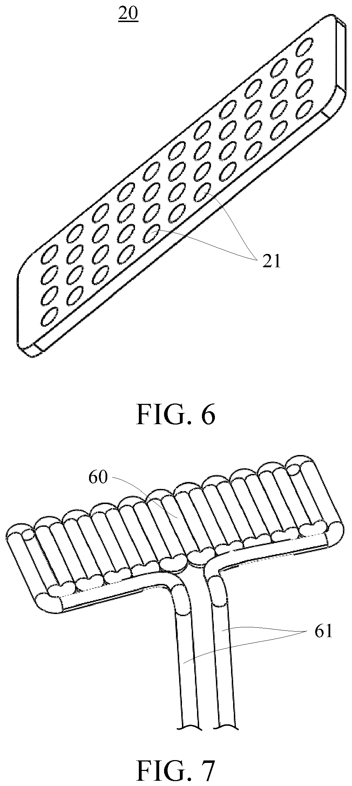

FIG. 5 and FIG. 6 are respectively a schematic three-dimensional exploded view of the aerosol supply system 100 shown in FIG. 1 and a schematic three-dimensional view of the induction heating body 20 of the aerosol supply system 100. As shown in the figures, in an optional embodiment, the induction heating body 20 is a flat extended heating sheet with holes. For example, the induction heating body 20 may be in a flat plate shape as a whole, and is provided with a plurality of through holes 21 in a thickness direction of the induction heating body 20, so that the aerosol generated through atomization can penetrate the through holes 21 and diverge in a direction away from the capillary element 30. The induction heating body 20 may be made by opening holes in a sheet material, or may use a heating mesh structure having meshes.

FIG. 7 is a schematic three-dimensional view of the electromagnetic induction coil 60 of the aerosol supply system 100. As shown in FIG. 7, in an optional embodiment, the electromagnetic induction coil 60 is a coil extending in a plane. The electromagnetic induction coil 60 may be bent to form a substantially square structure, and is in a flat plate shape as a whole. In addition, two ends of the electromagnetic induction coil 60 may be connected to a wire pin 61, to be conductively connected to a battery 80 in the power supply assembly 100B. The electromagnetic induction coil 60 is arranged in a plane extending shape, to facilitate placement and positioning of the electromagnetic induction coil 60.

In an optional embodiment, as shown in FIG. 2 to FIG. 4, the aerosol supply system 100 includes an airflow channel, and a part of the airflow channel guides airflow to bypass or penetrate the capillary element 30 and reach an atomization space 45A above the induction heating body 20. For example, the airflow channel as a whole may start at an air inlet end 72 of the aerosol supply system 100, and end at an air outlet end 18 of the aerosol supply system 100. The air inlet end 72 may be a through hole provided on a battery housing 70. The air outlet end 18 may be located at a mouthpiece portion 14 of a housing assembly 10. The airflow channel may be arranged so that after the airflow enters the aerosol supply system 100 through the air inlet end 72, the airflow is conveyed through a channel next to the capillary element 30, and then conveyed to the atomization space 45A above the induction heating body 20 through a channel higher than the capillary element 30. Alternatively, the airflow channel may be arranged so that after the airflow enters the aerosol supply system 100 through the air inlet end 72, the airflow is conveyed to the atomization space 45A above the induction heating body 20 through a channel located in the capillary element 30, which can shorten an airflow stroke. In this way, after the airflow enters the atomization space 45A, an aerosol generated through atomization in the atomization space 45A can be carried out of the air outlet end 18.

In an optional embodiment, as shown in FIG. 2 to FIG. 4, the capillary element 30 is positioned further away from the liquid storage cavity 10A than the induction heating body 20. For example, the liquid storage cavity 10 A may be defined through the housing assembly 10, the capillary element 30 is arranged at the bottom of the housing assembly 10, and the induction heating body 20 is arranged on a side of the capillary element 30 close to the liquid storage cavity 10A. In this way, it is still convenient to orient a heating surface of the capillary element 30 toward or close to a smoke exhaust pipe 13 of the aerosol supply system 100, so that the aerosol generated through atomization does not need to bypass the capillary element 30, but directly enters the smoke exhaust pipe 13.

In an optional embodiment, as shown in FIG. 2 to FIG. 4, the induction heating body 20 is stacked on an upper surface of the capillary element 30, and is pressed and fixed on the capillary element 30 through a support 40. For example, both the induction heating body 20 and the capillary element 30 may be arranged in a flat plate shape, so that the induction heating body 20 can be stacked on the upper surface of the capillary element 30. The support 40 may be mounted in the aerosol supply system 100, and press and fix the induction heating body 20 on the capillary element 30, to achieve a fixing effect on the induction heating body 20 and the capillary element 30. The induction heating body 20 does not need to completely cover the upper surface of the capillary element 30, so that an exposed part of the surface of the capillary element 30 can release the aerosol. In this way, assembly of the induction heating body 20 and the capillary element 30 is also simple.

In an optional embodiment, as shown in FIG. 2, FIG. 3, and FIG. 5, the atomizer 100A of the aerosol supply system 100 includes the induction heating body 20 and the capillary element 30, and may further include a housing assembly 10. The housing assembly 10 defines the liquid storage cavity 10A, and includes a mouthpiece portion 14, a bottom end portion 15, and a smoke exhaust pipe 13. The bottom end portion 15 is arranged opposite to the mouthpiece portion 14, and the smoke exhaust pipe 13 extends from the mouthpiece portion 14 toward the bottom end portion 15 in the housing assembly 10. The capillary element 30 is located in the housing assembly 10 and arranged on the bottom end portion 15, and the induction heating body 20 is positioned closer to the smoke exhaust pipe 13 than the capillary element 30. In this way, the aerosol generated through atomization can directly enter the smoke exhaust pipe 13.

In an optional embodiment, as shown in FIG. 2, FIG. 3, FIG. 4, and FIG. 5, an air inlet channel is provided on the housing assembly 10, and the air inlet channel bypasses or penetrates the capillary element 30, to guide the airflow to vicinity of the induction heating body 20. The air inlet channel may be the foregoing airflow channel, or may be a part of the airflow channel.

In an optional embodiment, as shown in FIG. 2 to FIG. 4, the capillary element 30 is located between the induction heating body 20 and the bottom end portion 15 of the housing assembly 10. For example, the capillary element 30 may be arranged at the bottom of the housing assembly 10, and the induction heating body 20 may be arranged on a side of the capillary element 30 close to the liquid storage cavity 10A. In this way, it is convenient to orient a heating surface of the capillary element 30 toward or close to a smoke exhaust pipe 13 of the aerosol supply system 100, so that the aerosol generated through atomization does not need to bypass the capillary element 30, but directly enters the smoke exhaust pipe 13.

In an optional embodiment, as shown in FIG. 2 to FIG. 4, the induction heating body 20 is pressed and fixed on the capillary element 30 through a support 40. The support 40 is located in the housing assembly 10, and defines a first channel 44 in communication with the liquid storage cavity 10A to guide the liquid substrate to the capillary element 30 and a second channel 45 in communication with the smoke exhaust pipe 13, and at least a part of the second channel 45 is used as an atomization space 45A above the induction heating body 20. In this way, pressing and fixing of the induction heating body 20 and the capillary element 30 by the support 40 can be implemented, and the first channel 44 and the second channel 45 for liquid and gas communication through the support 40 can also be provided. In addition, an end portion of the capillary element 30 may be located below the first channel 44, so that the capillary element 30 can absorb the liquid substrate in the first channel 44.

In an optional embodiment, as shown in FIG. 3 and FIG. 5, the support 40 includes an elastic pressing member 41 and a supporting component 42 configured to support the elastic pressing member 41. The elastic pressing member 41 is configured to press and fix the induction heating body 20 on a surface of the capillary element 30. In addition, the support 40 may further include a first sealing member 43. The first sealing member 43 is connected to an upper end of the supporting component 42, and the elastic pressing member 41 is connected to a lower end of the supporting component 42.

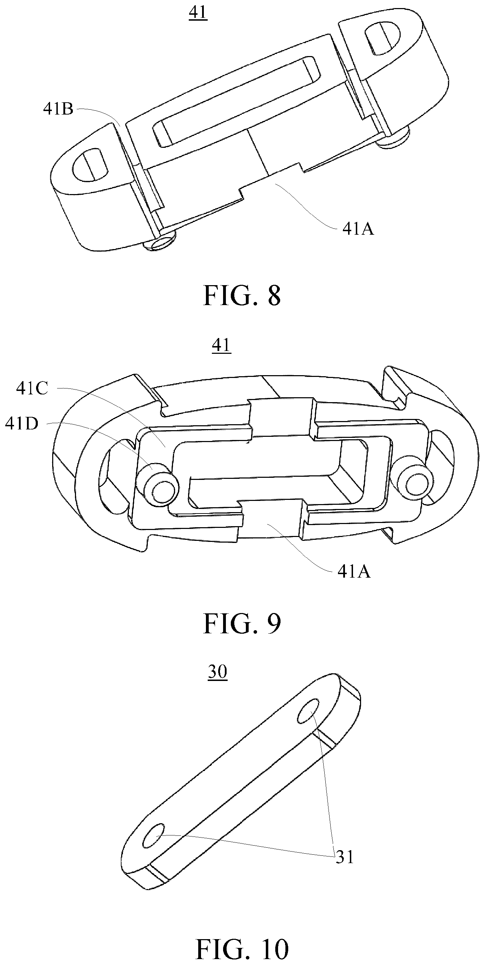

For example, FIG. 8 and FIG, 9 are respectively two schematic three-dimensional views of the elastic pressing member 41. A top end of the elastic pressing member 41 may include a clamping groove 41B, for clamping and matching with the supporting component 42; and a bottom end of the elastic pressing member 41 may include an abutting surface 41C and positioning posts 41D extending downward from the abutting surface 41C. The elastic pressing member 41 may press and fix the induction heating body 20 on the capillary element 30 through the abutting surface 41C. For example, the elastic pressing member 41 may be made of silica gel.

In addition, FIG. 10 is a schematic three-dimensional view of the capillary element 30. Positioning holes 31 may be provided at two ends of the capillary element 30, so that the positioning posts 41D of the elastic pressing member 41 can be inserted into the positioning holes 31 of the capillary element 30, to implement mounting and positioning of the capillary element 30.

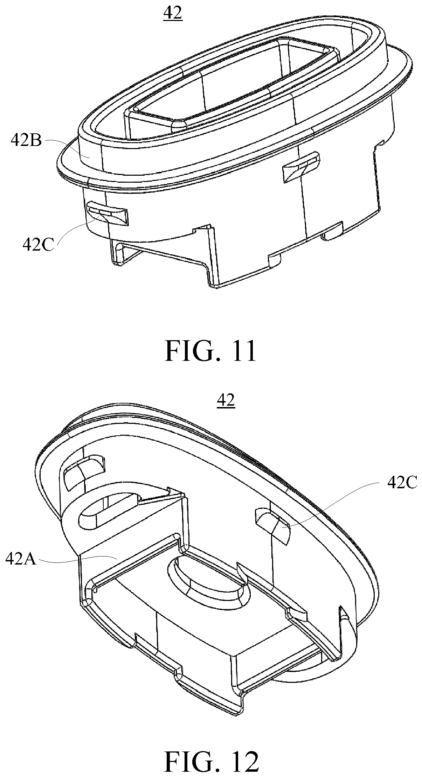

For example, FIG. 11 and FIG. 12 are respectively two schematic three-dimensional views of the supporting component 42. The supporting component 42 may be made of a hard material, to provide structural support, where the lower end of the supporting component 42 may include an insertion plate 42A, for matching with the clamping groove 41B of the elastic pressing member 41; and the upper end of the supporting component 42 may include an annular wall 42B, for sleeving the first sealing member 43 thereon. A clamping block 42C may further be provided on a side wall of the supporting component 42, for buckling and matching with the housing assembly 10.

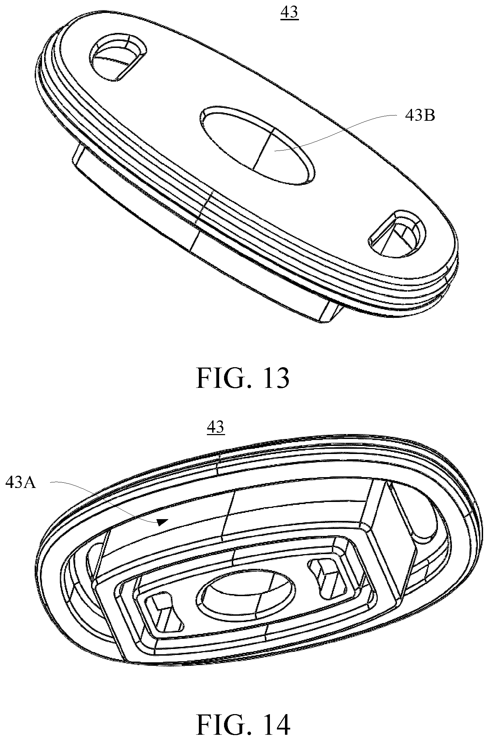

For example, FIG. 13 and FIG. 14 are respectively two three-dimensional views of the first sealing member 43. The first sealing member 43 may include an annular insertion groove 43A, the annular wall 42B is accommodated by the annular insertion groove 43A to mount the first sealing member 43 on the supporting component 42, and form sealing between the supporting component 42 and the housing assembly 10. The support 40 is arranged to include the elastic pressing member 41, the supporting component 42, and the first sealing member 43, so that elastic pressing of the induction heating body 20, necessary sealing of the liquid storage cavity 10A of the housing assembly 10, and overall structural strength of the support 40 can be implemented. In addition, an insertion hole 43B may be provided in the middle of the first sealing member 43, for insertion and matching of a lower end of the smoke exhaust pipe 13. For example, the first sealing member 43 may be made of silica gel.

In an optional embodiment, as shown in FIG. 2, FIG. 3, FIG. 5, and FIG. 15, FIG. 15 is a schematic three-dimensional view of a base 12 of the aerosol supply system 100 shown in FIG. 5. An air inlet hole 16 is provided on the bottom end portion 15 of the housing assembly 10, and the air inlet hole 16 extends in a longitudinal direction, so that a port of the air inlet hole 16 is higher than an upper surface of the capillary element 30. A notch 41A is provided on the support 40, so that airflow output through the air inlet hole 16 reaches the atomization space 45A above the induction heating body 20 via the notch 41A. For example, the housing assembly 10 may include an upper housing 11 and a base 12, and the base 12 includes the bottom end portion 15. The air inlet hole 16 may be provided on the base 12 of the housing assembly 10, where an upper end opening of the air inlet hole 16 is provided higher than the capillary element 30, and may further be higher than the induction heating body 20. The notch 41A may be provided on the elastic pressing member 41 of the support 40. In addition, a clamping groove 12A may be provided on a side wall of the base 12, for buckling and matching with the clamping block 42C on the side wall of the supporting component 42. Because there is no need to provide a channel of a wire or an electrode sheet, the base 12 may be directly provided with an upper opening accommodation groove for accommodating the capillary element 30, so that liquid leakage can be prevented.

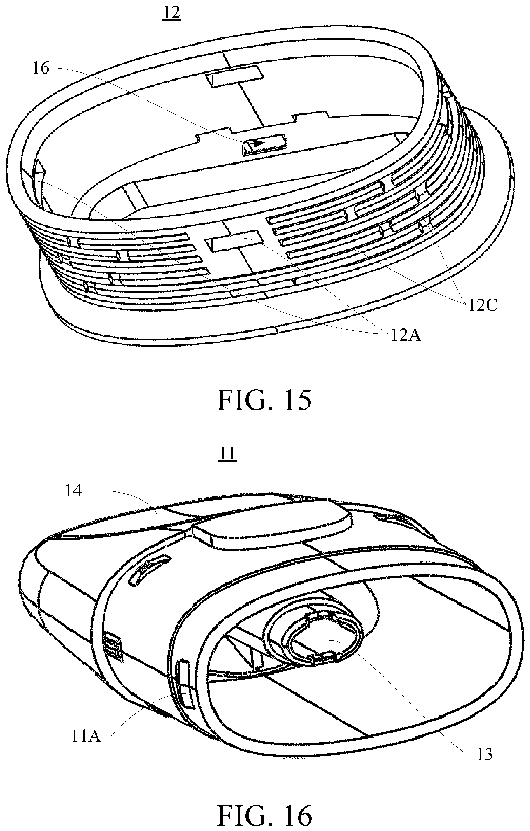

In addition, as shown in FIG. 15, a plurality of liquid leakage storage grooves 12C may further be formed on an outer side surface of the base 12. The liquid leakage storage grooves 12C may be provided concave from the outer side surface of the base 12, or may be defined by a plurality of convex strips provided on the outer side surface of the base 12. Through forming of the liquid leakage storage grooves 12C, liquid leaking to the outer side surface of the base 12 can be absorbed and stored through the liquid leakage storage grooves 12C, for example, through capillary action, to limit flow of the leaked liquid. As shown in FIG. 5, an annular groove 12D may further be formed on the outer side surface of the base 12, and a sealing ring 50 may be embedded in the annular groove 12D, to form sealing with the upper housing 11.

In an optional embodiment, as shown in FIG. 2, FIG. 3, FIG. 4, FIG. 5, and FIG. 16, FIG. 16 is a schematic three-dimensional view of an upper housing 11 of the aerosol supply system 100 shown in FIG. 5. The upper housing 11 is generally constructed into a hollow cylindrical shape, where the upper housing 11 has a mouthpiece portion 14 located at a near end and an opening at a far end, to facilitate assembly of various functional components in the upper housing 11 through the opening. A cross section of the far end may be a racetrack shape, an oval shape, or the like that is close to a flat shape. The liquid storage cavity 10A is mainly defined by the upper housing 11, and the smoke exhaust pipe 13 extends from the mouthpiece portion 14 toward the far end in the housing assembly 10. A clamping block 11A may be provided on an outer side of the upper housing 11 close to the far end.

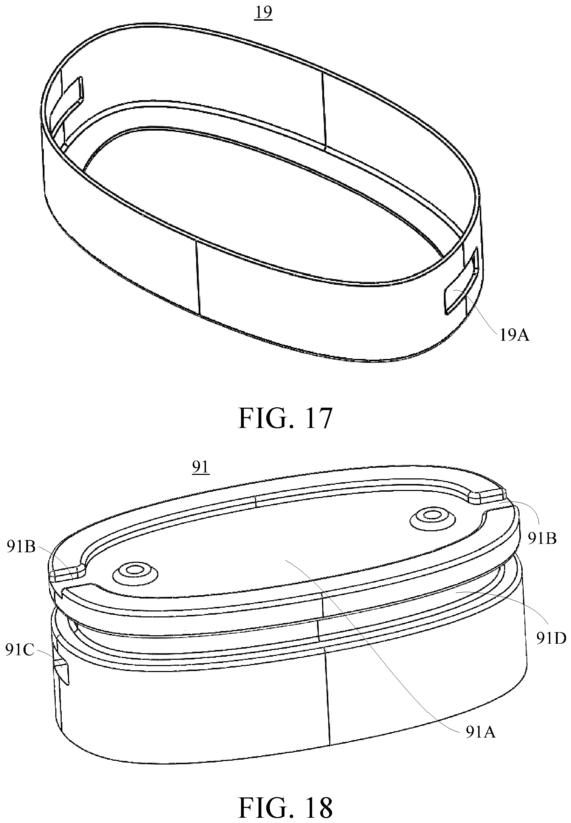

In an optional embodiment, as shown in FIG. 3, FIG. 4, FIG. 5, FIG. 16, and FIG. 17, FIG. 17 is a schematic three-dimensional view of a bottom cover 19 of the aerosol supply system 100 shown in FIG. 5. The bottom cover 19 may be made of metal, and a clamping hole 19A may be provided on a side wall of the bottom cover, for buckling and matching with the clamping block 11A of the upper housing 11, to keep the base 12 in combination with the upper housing 11.

In an optional embodiment, as shown in FIG. 1 and FIG. 2, the aerosol supply system 100 includes the power supply assembly 100B. The power supply assembly 100B includes a battery housing 70, a battery 80, and the electromagnetic induction coil 60, where the battery housing 70 accommodates the battery 80 and the electromagnetic induction coil 60, and the electromagnetic induction coil 60 is electrically connected to the battery 80. The electromagnetic induction coil 60 may be, for example, located above the battery 80. The battery 80 is configured to provide a current to the electromagnetic induction coil 60, so that the electromagnetic induction coil 60 generates the variable magnetic field. The electromagnetic induction coil 60 should be arranged as close as possible to the induction heating body 20 in the atomizer 100A, so that the induction heating body 20 is located in the electromagnetic field generated by the electromagnetic induction coil 60.

In an optional embodiment, as shown in FIG. 3, the electromagnetic induction coil 60 and the induction heating body 20 are arranged in parallel. The induction heating body 20 and the electromagnetic induction coil 60 are arranged in plane extending shapes, so that the induction heating body 20 and the electromagnetic induction coil 60 can be arranged in parallel, to facilitate placement and positioning of the induction heating body 20 and the electromagnetic induction coil 60.

In some other embodiments, the electromagnetic induction coil 60 may have another structure and arrangement. For example, the electromagnetic induction coil may be constructed into a three-dimensional cylindrical spiral coil, and the electromagnetic induction coil is arranged in a circumferential direction of an accommodation cavity of the power supply assembly 100B. When a cartridge is inserted into the accommodation cavity, the induction heating body 20 is exactly surrounded by the cylindrical spiral coil, so that the induction heating body 20 can be located in magnetic field space.

In an optional embodiment, as shown in FIG. 2 and FIG. 5, the battery housing 70 further defines an insertion cavity 71, and a part of the atomizer 100A may be inserted into the insertion cavity 71.

Further, to ensure induction heating generation efficiency, when the atomizer 100A in a form of the cartridge is placed into the insertion cavity 71, a distance between the flat induction heating body 20 and the electromagnetic induction coil 60 extending in a plane should be as small as possible, and considering that the capillary element 30 in the middle needs to have an oil storage capacity and an oil locking capability, the capillary element 30 cannot be too thin. For example, to ensure high heating efficiency, an effective distance between the induction heating body 20 and the electromagnetic induction coil 60 may range from 2 mm to 4 mm, and is preferably about 3 mm, and a thickness of the capillary element 30 may be controlled to range from 1 mm to 1.5 mm.

In an optional embodiment, as shown in FIG. 2, FIG. 3, FIG. 4, and FIG. 18, FIG. 18 is a schematic three-dimensional view of an insulation member 91 of the aerosol supply system 100 shown in FIG. 5. The insulation member 91 is arranged in the battery housing 70, configured to cover the electromagnetic induction coil 60. A concave portion 91A may be formed on a top end of the insulation member 91, and the concave portion 91A may be used as a part of the airflow channel in the aerosol supply system 100. Two sides of the concave portion 91A are in communication with grooves 91B, and the grooves 91B correspond to and are in communication with the air inlet end 72 provided on the battery housing 70. A clamping groove 91C and an annular groove 91D may further be arranged on a side wall of the insulation member 91. A sealing ring 94 may be embedded in the annular groove 91D, to form sealing with the battery housing 70, thereby preventing a liquid substrate seeping from the atomizer 100A to the insertion cavity 71 from flowing to a controller 95, a sensor 96, and other components in the power supply assembly 100B. The sensor 96 may be configured to sense inhalation airflow generated by the atomizer 100A during inhalation, so that the controller 95 controls, based on a detection signal of the sensor 96, the battery 80 to output a current to the electromagnetic induction coil 60.

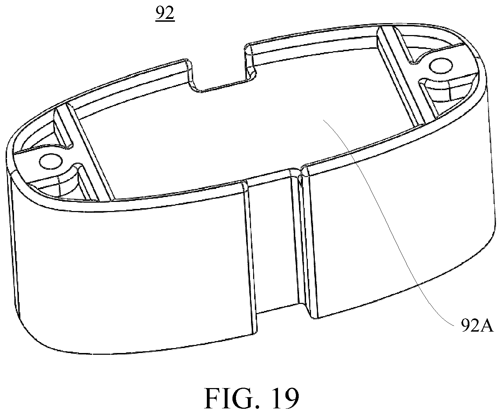

In an optional embodiment, as shown in FIG. 2, FIG. 3, and FIG. 19, FIG. 19 is a schematic three-dimensional view of a coil support 92 of the aerosol supply system 100. An accommodation groove 92A may be formed on a top end of the coil support 92, for placing the electromagnetic induction coil 60 therein. Again, as shown in FIG. 2, the power supply assembly 100B may further include a mounting support 93, where the mounting support 93 may accommodate and hold the battery 80, and may hold the coil support 92 and the electromagnetic induction coil 60 on the mounting support 93 through buckling and matching between a buckle and the clamping groove 91C of the insulation member 91.

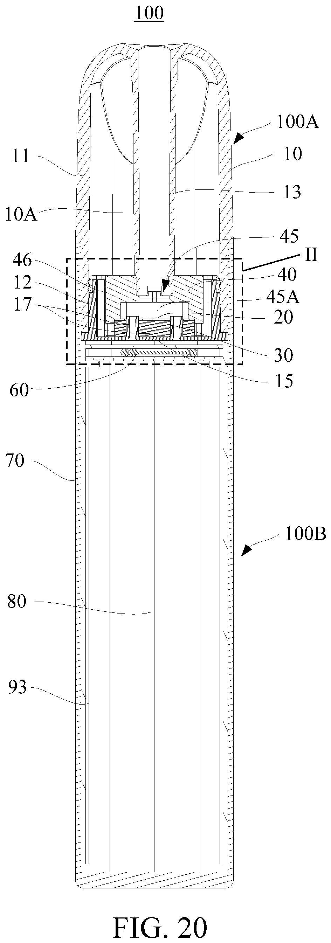

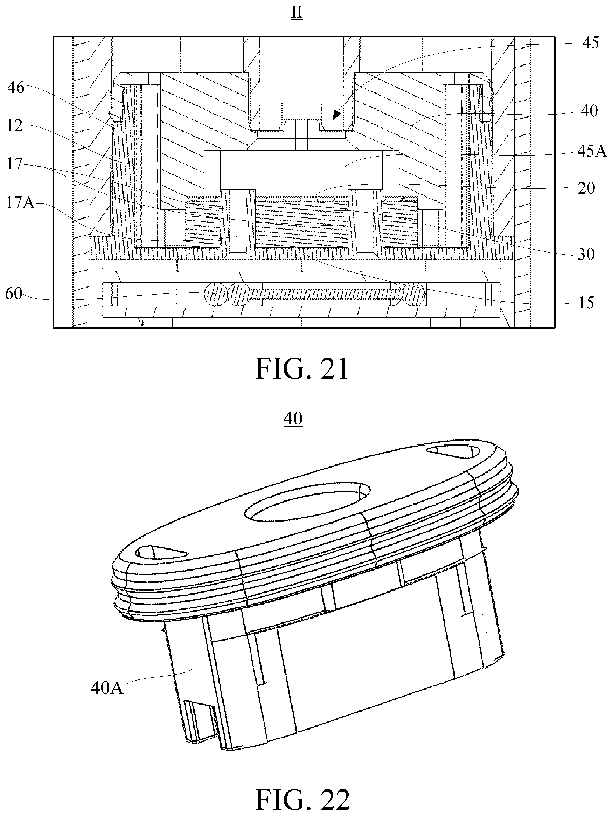

FIG. 20 and FIG. 21 are respectively a schematic sectional view and a schematic partial enlarged view of an aerosol supply system 100 according to another embodiment of this application. The aerosol supply system 100 in this embodiment is substantially the same as the foregoing aerosol supply system, and also includes an atomizer 100A and a power supply assembly 100B. The atomizer 100A further includes a housing assembly 10, an induction heating body 20, a capillary element 30, and a support 40. The power supply assembly 100B further includes an electromagnetic induction coil 60, a battery housing 70, and a battery (where this is not shown in the figure, and may refer to the battery 80 in FIG. 2).

A difference between the aerosol supply system 100 in this embodiment and the foregoing aerosol supply system is that: in the aerosol supply system 100 in the embodiment shown in FIG. 20, a bottom end portion 15 of the housing assembly 10 includes an air inlet post 17 extending through the capillary element 30, and a tail end of the air inlet post 17 is higher than an upper surface of the capillary element 30. The interior of the air inlet post 17 is hollow, to provide a vertical air guiding channel 17A. Through arrangement of the air inlet post 17 in this way, airflow can be directly conveyed upward and enter an atomization space 45A above the induction heating body 20, which can shorten an airflow stroke. An end surface of the end of the air inlet post 17 is higher than an atomization surface of the capillary element 30, so that e-liquid can be prevented from leaking.

In some embodiments, a quantity of air inlet posts 17 may be one or more, for example, may be two. An axis of the air inlet post 17 and an axis of a smoke exhaust pipe 13 of the housing assembly 10 may be arranged to be staggered, to prevent condensate in the smoke exhaust pipe 13 from falling down on the air inlet post 17 and causing blockage.

In some other embodiments, an end surface of the air inlet post 17 may be designed to be closed, and an air outlet hole in a radial direction may be provided on a side portion of the air inlet post 17 to be in communication with the vertical air guiding channel 17A. The air outlet hole is provided to be not covered by the capillary element 30, for example, oil guiding cotton, and the induction heating body 20, for example, a heating sheet, and may be close to a surface of the induction heating body 20.

In some other embodiments, the support 40 may define a second channel 45 in communication with the smoke exhaust pipe 13, where an inner surface of the second channel 45 close to the air inlet post 17 may have an inclined surface for guiding the airflow from two sides of the atomization space 45A to the smoke exhaust pipe 13 in the middle.

In some embodiments, as shown in FIG. 20, FIG. 22, and FIG. 23, FIG. 22 and FIG. 23 are respectively two schematic three-dimensional views of the support 40 of the aerosol supply system 100 shown in FIG. 20. The support 40 may include an abutting surface 41C. Therefore, the induction heating body 20 can be pressed and fixed on the capillary element 30 through the abutting surface 41C of the support 40. The support 40 is a second sealing member, for example, the support 40 may be made of silica gel, to have supporting and sealing properties.

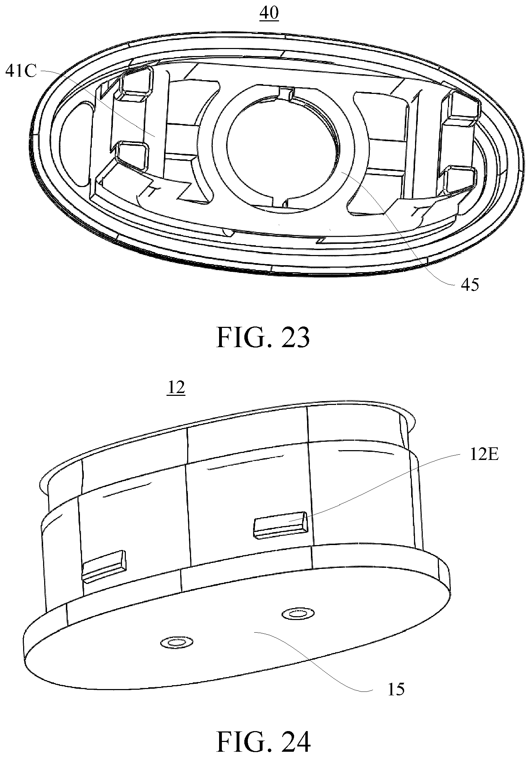

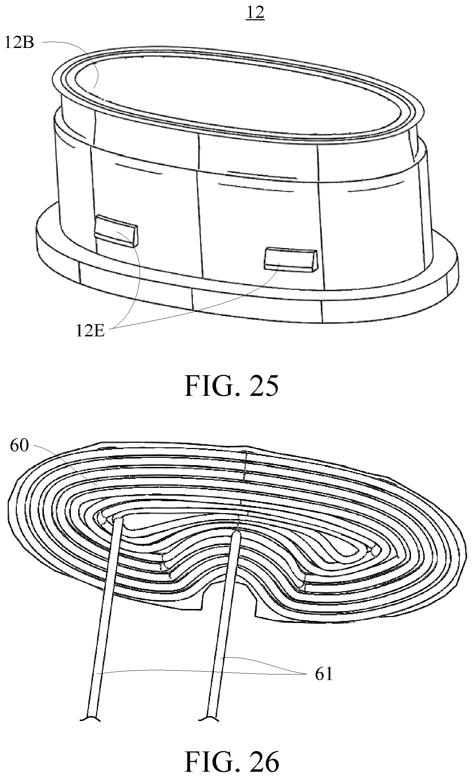

The housing assembly 10 may include an upper housing 11 and a base 12. FIG. 24 and FIG. 25 are respectively two three-dimensional views of the base 12 of the aerosol supply system 100 shown in FIG. 20. The base 12 includes the bottom end portion 15. The support 40 used as the second sealing member is mounted on the base 12; and the base 12 may be inserted into a lower end opening of the upper housing 11. At least a part of the support 40 used as the second sealing member is constructed to provide sealing between the base 12 and the upper housing 11. A side wall 40A of the second sealing member and a side wall 12B of the base 12 enclose a third channel 46, the third channel 46 is in communication with a liquid storage cavity 10A to guide a liquid substrate to the capillary element 30, the second sealing member further includes a second channel 45, and at least a part of the second channel 45 is constructed as the atomization space 45A above the induction heating body 20. By using an integrated support 40, a quantity of components can be reduced, thereby facilitating assembly. In addition, a clamping block 12E may further be provided on the side wall of the base 12, for buckling and matching with the upper housing 11.

FIG. 26 is a schematic three-dimensional view of the electromagnetic induction coil 60 of the aerosol supply system 100 shown in FIG. 20. As shown in the figure, in an optional embodiment, the electromagnetic induction coil 60 is a spiral coil extending in a plane. The electromagnetic induction coil 60 may be wound or spiraled to form a substantially circular or oval structure, and is in a flat plate shape as a whole. In addition, two ends of the electromagnetic induction coil 60 may be connected to a wire pin 61, to be conductively connected to the battery in the power supply assembly 100B. The electromagnetic induction coil 60 is arranged in a plane extending shape, to facilitate placement and positioning of the electromagnetic induction coil 60.

Various components of an aerosol supply system 100 and an atomizer 100A thereof in this application are described above. When an aerosol supply system 100 needs to be used for inhalation, first, a power switch of a power supply assembly 100B may be turned on, so that a battery 80 supplies power to an electromagnetic induction coil 60; and then, when a user performs inhalation on a mouthpiece portion 14 of an atomizer 100A, a controller 95 of the aerosol supply system 100 may start the power supply assembly 100B and the atomizer 100 A to operate based on an inhalation action, to generate an aerosol for the user to inhale at last. A liquid substrate from a liquid storage cavity 10A is heated and atomized through an induction heating body 20 to form the aerosol, external air may sequentially flow through an air inlet end 72, a groove 91B, a concave portion 91A, an air inlet hole 16, and a notch 41A (or an air guiding channel 17A), to be conveyed above an atomization surface of a capillary element 30, and the formed aerosol is carried out of a smoke exhaust pipe 13 through a second channel 45.

Finally, it should be noted that, the foregoing embodiments are merely used for describing the technical solutions of this application, but are not intended to limit this application. Under the concept of this application, technical features in the foregoing embodiments or different embodiments may be combined, steps may be implemented in any sequence, and there may be many other changes in different aspects of this application as described above. For brevity, those are not provided in detail. Although this application is described in detail with reference to the foregoing embodiments, a person of ordinary skill in the art should understand that, they may still make modifications to the technical solutions described in the foregoing embodiments or make equivalent substitutions to some technical features thereof, and the modifications or substitutions do not make the nature of corresponding technical solutions depart from the scope of the technical solutions in the embodiments of this application.

## Claims

1. An aerosol supply system, configured to atomize a liquid substrate to generate an aerosol, wherein the aerosol supply system comprises:
a liquid storage cavity, for storing the liquid substrate;
an electromagnetic induction coil, configured to generate a variable magnetic field;
an induction heating body, configured to induce heating in the variable magnetic field; and
a capillary element, arranged in contact with the induction heating body, and configured to receive and store the liquid substrate from the liquid storage cavity and convey the liquid substrate to vicinity of the induction heating body, wherein the capillary element is positioned between the electromagnetic induction coil and the induction heating body.

2. The aerosol supply system according to claim 1, wherein
the induction heating body is a flat extended heating sheet with holes; and/or
the electromagnetic induction coil is a coil extending in a plane.

3. The aerosol supply system according to claim 1, wherein
the aerosol supply system comprises an airflow channel, and a part of the airflow channel guides airflow to bypass or penetrate the capillary element and reach an atomization space above the induction heating body.

4. The aerosol supply system according to claim 1, wherein
the capillary element is positioned further away from the liquid storage cavity than the induction heating body.

5. The aerosol supply system according to claim 1, wherein
the induction heating body is stacked on an upper surface of the capillary element, and is pressed and fixed on the capillary element through a support.

6. The aerosol supply system according to claim 1, wherein
the aerosol supply system comprises a housing assembly, the housing assembly defines the liquid storage cavity and comprises a mouthpiece portion, a bottom end portion, and a smoke exhaust pipe, the bottom end portion is arranged opposite to the mouthpiece portion, and the smoke exhaust pipe extends from the mouthpiece portion toward the bottom end portion in the housing assembly; and
the capillary element is located in the housing assembly and arranged on the bottom end portion, and the induction heating body is positioned closer to the smoke exhaust pipe than the capillary element.

7. The aerosol supply system according to claim 6, wherein
the aerosol supply system further comprises a support located in the housing assembly, the support defines a first channel in communication with the liquid storage cavity to guide the liquid substrate to the capillary element and a second channel in communication with the smoke exhaust pipe, and at least a part of the second channel is used as an atomization space above the induction heating body.

8. The aerosol supply system according to claim 7, wherein
the support comprises an elastic pressing member and a supporting component configured to support the elastic pressing member; and
the elastic pressing member is configured to press and fix the induction heating body on a surface of the capillary element.

9. The aerosol supply system according to claim 7, wherein
an air inlet hole is provided on the bottom end portion of the housing assembly, and the air inlet hole extends in a longitudinal direction, so that a port of the air inlet hole is higher than an upper surface of the capillary element; and
a notch is provided on the support, so that airflow output through the air inlet hole reaches the atomization space above the induction heating body via the notch.

10. The aerosol supply system according to claim 6, wherein
the bottom end portion of the housing assembly comprises an air inlet post extending through the capillary element, and the interior of the air inlet post is hollow, and a tail end of the air inlet post is higher than an upper surface of the capillary element.

11. The aerosol supply system according to claim 6, wherein
the induction heating body is pressed and fixed on the capillary element through a support;
the housing assembly comprises an upper housing and a base, the base comprises the bottom end portion, the support is mounted on the base and is used as a second sealing member, and at least a part of the second sealing member is constructed to provide sealing between the base and the upper housing; and
the second sealing member and the base enclose a third channel, the third channel is in communication with the liquid storage cavity to guide the liquid substrate to the capillary element, the second sealing member further comprises a second channel in communication with the smoke exhaust pipe, and at least a part of the second channel is constructed as an atomization space above the induction heating body.

12. The aerosol supply system according to any one of claims 1 to 11, wherein
the aerosol supply system comprises a power supply assembly, the power supply assembly comprises a battery housing, a battery, and the electromagnetic induction coil, the battery housing accommodates the battery and the electromagnetic induction coil, and the electromagnetic induction coil is electrically connected to the battery.

13. The aerosol supply system according to claim 12, wherein
the electromagnetic induction coil and the induction heating body are arranged in parallel.

14. An atomizer of an aerosol supply system, wherein the atomizer comprises:
a housing assembly, defining a liquid storage cavity for storing a liquid substrate, and comprising a mouthpiece portion, a bottom end portion, and a smoke exhaust pipe, wherein the bottom end portion is arranged opposite to the mouthpiece portion, and the smoke exhaust pipe extends from the mouthpiece portion toward the bottom end portion in the housing assembly;
an induction heating body, configured to be capable of inducing heating in a variable magnetic field, to heat a part of the liquid substrate to generate an aerosol; and
a capillary element, arranged in contact with the induction heating body, and configured to receive and store the liquid substrate from the liquid storage cavity and convey the liquid substrate to vicinity of the induction heating body, wherein the induction heating body is positioned closer to the smoke exhaust pipe than the capillary element.

15. The atomizer according to claim 14, wherein
the capillary element is located between the induction heating body and the bottom end portion of the housing assembly.

16. The atomizer according to claim 14, wherein
the induction heating body is stacked on an upper surface of the capillary element, and is pressed and fixed on the capillary element through a support.

17. The atomizer according to claim 14, wherein
the capillary element is arranged in a flat plate shape, and a thickness of the capillary element ranges from 1 mm to 1.5 mm.

18. The atomizer according to any one of claims 14 to 17, wherein
an air inlet channel is provided on the housing assembly, and the air inlet channel bypasses or penetrates the capillary element, to guide airflow to vicinity of the induction heating body.
